# EUROPEAN PATENT APPLICATION

(11) **EP 2 124 055 A1**
(43) Date of publication of application: **25.11.2009**
(21) Application number: 08711714.9
(22) Date of filing: 21.02.2008
(51) Int. Cl.: G01N 33/53

(54) **KIT FOR IMMUNOASSAY OF BSH, AND METHOD FOR MEASUREMENT OF BSH**

(30) Priority: 22.02.2007 JP 2007042428
(71) Applicant: Stella Chemifa Corporation, Osaka-shi Osaka 541-0047 (JP)
(72) Inventor: KIRIHATA, Mitsunori, Sakai-shi Osaka 599-8231 (JP); ASANO, Tomoyuki, Sakai-shi Osaka 591-8025 (JP); UEHARA, Kohki, Sakai-shi Osaka 591-8025 (JP)
(74) Representative: Vossius & Partner
(86) International application number: PCT/JP2008/052925
(87) International publication number: WO 2008/102833

(57) **Abstract**

To highly sensitively and easily measure BSH in a bodily fluid. There is provided a measuring method for BSH in a bodily fluid including: 1) a step of diluting a taken bodily fluid with a diluent for a bodily fluid, and contacting the obtained diluted sample solution with an antibody-immobilized member wherein an anti-BSH monoclonal antibody or a fragment thereof is immobilized to a carrier in the presence of a conjugate of a hapten of BSH and a substance having discrimination capability; and 2) a step of detecting the amount of an immunocomplex of BSH in the bodily fluid and the anti-BSH monoclonal antibody or the fragment thereof, which can be formed by the contacting; and a kit for carrying out the method.

SEQUENCE LISTING

<110> STELLA CHEMIFA CORPORATION

<120> KIT FOR IMMUNOASSAY MEASUREMENT OF BSH AND A METHOD FOR MEASUREMENT OF BSH

<130> PCT07030SF

<160> 4

<170> PatentIn version 3.1

<210> 1
<211> 657
<212> DNA
<213> mouse

<400> 1
ctcgagtctg gccctggaat attgcagcgc tcccagaccc tcagtctgac ttgttctttc 60

tctgggtttt cactgagcac ttctggtatg ggtgttggct ggtttcgtca gccttcaaca 120

aagggtctag agtggctggc agacatttgg tggaatgaca ataaatacta taatccatcc 180

ctgaagagcc ggctcacaat ctccaaggat acctccaaaa accaggtatt cctcaagatc 240

gccagtgtgg acactataga tactgccact tactactgtt ctctaagaaa tagtgccgaa 300

aagacaaaca cctggggcca aggcaccact ctcacagtct cctcagccaa aacgacaccc 360

ccatctgtct atccactggc ccctggatct gctgcccaaa ctaactccat ggtgaccctg 420

ggatgcctgg tcaagggcta tttccctgag ccagtgacag tgacctggaa ctctggatcc 480

ctgtccagcg gtgtgcacac cttcccagct gtcctgcagt ctgacctcta cactctgagc 540

agctcagtga ctgtcccctc cagcacctgg cccagcgaga ccgtcacctg caacgttgcc 600

cacccggcca gcagcaccaa ggtggacaag aaaattgtgc ccagggattg tactagt 657

<210> 2
<211> 655
<212> DNA
<213> mouse

<400> 2
gagctcgttg tgactcagga atctgcactc accacatcac ctggtgaaac agtcacactc 60

acttgtcgct caagtactgg ggctgttaca actagtaact atgtcaattg ggtccaagaa 120

aaaccagatc atttattcac tggtctaata ggtggtacca acaaccgagc tccaggtgtt 180

cctgccagat tctcaggctc cctgattgga gacaaggctg ccctcaccat cacaggggca 240

cagactgagg atgaggcaat atatttctgt ggtctatggt acagcaacca ttgggtgttc 300

ggtggaggaa ccaaactgac tgtcctaggc cagcccaagt cttcgccatc agtcaccctg 360

tttccacctt cctctgaaga gctcgagact aacaaggcca cactggtgtg tacgatcact 420

gatttctacc caggtgtggt gacagtggac tggaaggtag atggtacccc tgtcactcag 480

ggtatggaga caacccagcc ttccaaacag agcaacaaca agtacatggc tagcagctac 540

ctgaccctga cagcaagagc atgggaaagg catagcagtt acagctgcca ggtcactcat 600

gaaggtcaca ctgtggagaa gagtttgtcc cgtgctgagt gttcctaatt ctaga 655

<210> 3
<211> 219
<212> PRT
<213> mouse

<400> 3

Leu Glu Ser Gly Pro Gly Ile Leu Gln Arg Ser Gln Thr Leu Ser Leu
1               5                   10                  15

Thr Cys Ser Phe Ser Gly Phe Ser Leu Ser Thr Ser Gly Met Gly Val
            20                  25                  30

Gly Trp Phe Arg Gln Pro Ser Thr Lys Gly Leu Glu Trp Leu Ala Asp
        35                  40                  45

Ile Trp Trp Asn Asp Asn Lys Tyr Tyr Asn Pro Ser Leu Lys Ser Arg
    50                  55                  60

Leu Thr Ile Ser Lys Asp Thr Ser Lys Asn Gln Val Phe Leu Lys Ile
65                  70                  75                  80

Ala Ser Val Asp Thr Ile Asp Thr Ala Thr Tyr Tyr Cys Ser Leu Arg
                85                  90                  95

Asn Ser Ala Glu Lys Thr Asn Thr Trp Gly Gln Gly Thr Thr Leu Thr
            100                 105                 110

Val Ser Ser Ala Lys Thr Thr Pro Pro Ser Val Tyr Pro Leu Ala Pro
        115                 120                 125

Gly Ser Ala Ala Gln Thr Asn Ser Met Val Thr Leu Gly Cys Leu Val
    130                 135                 140

Lys Gly Tyr Phe Pro Glu Pro Val Thr Val Thr Trp Asn Ser Gly Ser
145                 150                 155                 160

Leu Ser Ser Gly Val His Thr Phe Pro Ala Val Leu Gln Ser Asp Leu
                165                 170                 175

Tyr Thr Leu Ser Ser Ser Val Thr Val Pro Ser Ser Thr Trp Pro Ser
            180                 185                 190

Glu Thr Val Thr Cys Asn Val Ala His Pro Ala Ser Ser Thr Lys Val
        195                 200                 205

Asp Lys Lys Ile Val Pro Arg Asp Cys Thr Ser
    210                 215

<210> 4
<211> 215
<212> PRT
<213> mouse

<400> 4

Glu Leu Val Val Thr Gln Glu Ser Ala Leu Thr Thr Ser Pro Gly Glu
1               5                   10                  15

Thr Val Thr Leu Thr Cys Arg Ser Ser Thr Gly Ala Val Thr Thr Ser
            20                  25                  30

Asn Tyr Val Asn Trp Val Gln Glu Lys Pro Asp His Leu Phe Thr Gly
        35                  40                  45

Leu Ile Gly Gly Thr Asn Asn Arg Ala Pro Gly Val Pro Ala Arg Phe
    50                  55                  60

Ser Gly Ser Leu Ile Gly Asp Lys Ala Ala Leu Thr Ile Thr Gly Ala
65                  70                  75                  80

Gln Thr Glu Asp Glu Ala Ile Tyr Phe Cys Gly Leu Trp Tyr Ser Asn
                85                  90                  95

His Trp Val Phe Gly Gly Gly Thr Lys Leu Thr Val Leu Gly Gln Pro
            100                 105                 110

Lys Ser Ser Pro Ser Val Thr Leu Phe Pro Pro Ser Ser Glu Glu Leu
        115                 120                 125

Glu Thr Asn Lys Ala Thr Leu Val Cys Thr Ile Thr Asp Phe Tyr Pro
    130                 135                 140

Gly Val Val Thr Val Asp Trp Lys Val Asp Gly Thr Pro Val Thr Gln
145                 150                 155                 160

Gly Met Glu Thr Thr Gln Pro Ser Lys Gln Ser Asn Asn Lys Tyr Met
                165                 170                 175

Ala Ser Ser Tyr Leu Thr Leu Thr Ala Arg Ala Trp Glu Arg His Ser
            180                 185                 190

Ser Tyr Ser Cys Gln Val Thr His Glu Gly His Thr Val Glu Lys Ser
        195                 200                 205

Leu Ser Arg Ala Glu Cys Ser
    210                 215

SEQUENCE LISTING

<110> STELLA CHEMIFA CORPORATION

<120> KIT FOR IMMUNOASSAY MEASUREMENT OF BSH AND A METHOD FOR MEASUREMENT OF BSH

<130> PCT07030SF

<160> 4

<170> PatentIn version 3.1

<210> 1
<211> 657
<212> DNA
<213> mouse

<400> 1
ctcgagtctg gccctggaat attgcagcgc tcccagaccc tcagtctgac ttgttctttc 60

tctgggtttt cactgagcac ttctggtatg ggtgttggct ggtttcgtca gccttcaaca 120

aagggtctag agtggctggc agacatttgg tggaatgaca ataaatacta taatccatcc 180

ctgaagagcc ggctcacaat ctccaaggat acctccaaaa accaggtatt cctcaagatc 240

gccagtgtgg acactataga tactgccact tactactgtt ctctaagaaa tagtgccgaa 300

aagacaaaca cctggggcca aggcaccact ctcacagtct cctcagccaa aacgacaccc 360

ccatctgtct atccactggc ccctggatct gctgcccaaa ctaactccat ggtgaccctg 420

ggatgcctgg tcaagggcta tttccctgag ccagtgacag tgacctggaa ctctggatcc 480

ctgtccagcg gtgtgcacac cttcccagct gtcctgcagt ctgacctcta cactctgagc 540

agctcagtga ctgtcccctc cagcacctgg cccagcgaga ccgtcacctg caacgttgcc 600

cacccggcca gcagcaccaa ggtggacaag aaaattgtgc ccagggattg tactagt 657

<210> 2
<211> 655
<212> DNA
<213> mouse

<400> 2
gagctcgttg tgactcagga atctgcactc accacatcac ctggtgaaac agtcacactc 60

acttgtcgct caagtactgg ggctgttaca actagtaact atgtcaattg ggtccaagaa 120

aaaccagatc atttattcac tggtctaata ggtggtacca acaaccgagc tccaggtgtt 180

cctgccagat tctcaggctc cctgattgga gacaaggctg ccctcaccat cacaggggca 240

cagactgagg atgaggcaat atatttctgt ggtctatggt acagcaacca ttgggtgttc 300

ggtggaggaa ccaaactgac tgtcctaggc cagcccaagt cttcgccatc agtcaccctg 360

tttccacctt cctctgaaga gctcgagact aacaaggcca cactggtgtg tacgatcact 420

gatttctacc caggtgtggt gacagtggac tggaaggtag atggtacccc tgtcactcag 480

ggtatggaga caacccagcc ttccaaacag agcaacaaca agtacatggc tagcagctac 540

ctgaccctga cagcaagagc atgggaaagg catagcagtt acagctgcca ggtcactcat 600

gaaggtcaca ctgtggagaa gagtttgtcc cgtgctgagt gttcctaatt ctaga 655

<210> 3
<211> 219
<212> PRT
<213> mouse

<400> 3

Leu Glu Ser Gly Pro Gly Ile Leu Gln Arg Ser Gln Thr Leu Ser Leu
1               5                   10                  15

Thr Cys Ser Phe Ser Gly Phe Ser Leu Ser Thr Ser Gly Met Gly Val
            20                  25                  30

Gly Trp Phe Arg Gln Pro Ser Thr Lys Gly Leu Glu Trp Leu Ala Asp
        35                  40                  45

Ile Trp Trp Asn Asp Asn Lys Tyr Tyr Asn Pro Ser Leu Lys Ser Arg
    50                  55                  60

Leu Thr Ile Ser Lys Asp Thr Ser Lys Asn Gln Val Phe Leu Lys Ile
65                  70                  75                  80

Ala Ser Val Asp Thr Ile Asp Thr Ala Thr Tyr Tyr Cys Ser Leu Arg
                85                  90                  95

Asn Ser Ala Glu Lys Thr Asn Thr Trp Gly Gln Gly Thr Thr Leu Thr
            100                 105                 110

Val Ser Ser Ala Lys Thr Thr Pro Pro Ser Val Tyr Pro Leu Ala Pro
        115                 120                 125

Gly Ser Ala Ala Gln Thr Asn Ser Met Val Thr Leu Gly Cys Leu Val
    130                 135                 140

Lys Gly Tyr Phe Pro Glu Pro Val Thr Val Thr Trp Asn Ser Gly Ser
145                 150                 155                 160

Leu Ser Ser Gly Val His Thr Phe Pro Ala Val Leu Gln Ser Asp Leu
                165                 170                 175

Tyr Thr Leu Ser Ser Ser Val Thr Val Pro Ser Ser Thr Trp Pro Ser
            180                 185                 190

Glu Thr Val Thr Cys Asn Val Ala His Pro Ala Ser Ser Thr Lys Val
        195                 200                 205

Asp Lys Lys Ile Val Pro Arg Asp Cys Thr Ser
    210                 215

<210> 4
<211> 215
<212> PRT
<213> mouse

<400> 4

Glu Leu Val Val Thr Gln Glu Ser Ala Leu Thr Thr Ser Pro Gly Glu
1               5                   10                  15

Thr Val Thr Leu Thr Cys Arg Ser Ser Thr Gly Ala Val Thr Thr Ser
            20                  25                  30

Asn Tyr Val Asn Trp Val Gln Glu Lys Pro Asp His Leu Phe Thr Gly
        35                  40                  45

Leu Ile Gly Gly Thr Asn Asn Arg Ala Pro Gly Val Pro Ala Arg Phe
    50                  55                  60

Ser Gly Ser Leu Ile Gly Asp Lys Ala Ala Leu Thr Ile Thr Gly Ala
65                  70                  75                  80

Gln Thr Glu Asp Glu Ala Ile Tyr Phe Cys Gly Leu Trp Tyr Ser Asn
                85                  90                  95

His Trp Val Phe Gly Gly Gly Thr Lys Leu Thr Val Leu Gly Gln Pro
            100                 105                 110

Lys Ser Ser Pro Ser Val Thr Leu Phe Pro Pro Ser Ser Glu Glu Leu
        115                 120                 125

Glu Thr Asn Lys Ala Thr Leu Val Cys Thr Ile Thr Asp Phe Tyr Pro
    130                 135                 140

Gly Val Val Thr Val Asp Trp Lys Val Asp Gly Thr Pro Val Thr Gln
145                 150                 155                 160

Gly Met Glu Thr Thr Gln Pro Ser Lys Gln Ser Asn Asn Lys Tyr Met
                165                 170                 175

Ala Ser Ser Tyr Leu Thr Leu Thr Ala Arg Ala Trp Glu Arg His Ser
            180                 185                 190

Ser Tyr Ser Cys Gln Val Thr His Glu Gly His Thr Val Glu Lys Ser
        195                 200                 205

Leu Ser Arg Ala Glu Cys Ser
    210                 215

## Description

### TECHNICAL FIELD

The present invention relates to an immunoassay and kit for such immunoassay with the use of a monoclonal antibody against mercaptoundecahydrododecaborate (BSH, Borocaptate) or a fragment thereof. More specifically, the present invention relates to an immunoassay and kit for such immunoassay for BSH, which is useful particularly for detection and quantification of a neutron capture therapeutic agent used in boron-neutron capture therapy (BNCT).

### BACKGROUND ART

In recent years, boron-neutron capture therapy (BNCT) attracts attention as a new therapeutic method for cancer by utilizing a radioisotope. In boron-neutron capture therapy, a boron compound containing a ¹⁰boron isotope (¹⁰B) is incorporated into a cancer cell, which is then irradiated with a low-energy neutron ray (for example, thermal neutron) to distruct the cancer cell locally by a nuclear reaction occurring in the cell. In this therapeutic method, the selective accumulation of a ¹⁰B-containing boron compound in cells of cancer tissue is critical in enhancing the therapeutic effect, thus it is necessary to develop boron compounds that is incorporated selectively into cancer cells.

Conventionally, boron-containing compounds having a boron atom or a boron atomic group introduced into their basic skeleton have been synthesized as drugs used in BNCT. Clinically used drugs include p-boronophenylalanine (BPA) and mercaptoundecahydrododecaborate (BSH). Among these drugs, BSH is used mainly in treatment of a brain tumor in the form of a sodium salt and confirmed to be useful (see, for example, Non-Patent Documents 1 to 8).

In order to augment effectiveness of the treatment, it is desirable to accurately figure out the behavior of BSH in a living organism accompanying such BNCT, especially microdistribution in a cell surface or a cell, and confirm accumulation of BSH in a diseased site, to radiate neutron at an opportune time.

Non-Document 1: I. M. Wyzlic et al., Tetrahedron Lett., 1992, 33, 7489-7490.
Non-Document 2: W. Tjark, J. Organomet. Chem., 2000, 614-615, 37-47.
Non-Document 3: K. Imamura et al., Bull. Chem. Soc. Jpn., 1997, 70, 3103-3110.
Non-Document 4: A. S. Al-Madhorn et al., J. Med. Chem., 2002, 45, 4018-4028.
Non-Document 5: F. Compostella et al., Res. Develop. Neutron Capture Ther., 2002, 81-84.
Non-Document 6: S. B. Kahl et al., Progress in Neutron Capture Therapy for Cancer, Plenum Press, New York 1992, 223.
Non-Document 7: J. Cai et al., J. Med. Chem., 1997, 40, 3887-3896.
Non-Document 8: H. Lim et al., Res. Develop. Neutron Capture Ther., 2002, 37-42.

### DISCLOSURE OF THE INVENTION

### PROBLEM TO BE SOLVED BY THE INVENTION

However, details on behavior of BSH in a living organism accompanying BNCT, especially microdistribution in a cell surface or a cell are yet to be revealed, and development of a method which can easily and rapidly determine qualitatively and quantitatively behavior of BSH in a living organism is eagerly desired. There is also a problem that it is difficult to detect BSH distinguishing from BPA, which is used concurrently for therapy. Although an immunological measuring method is expected as a method for detection and quantitative determination of BSH, a low-molecular-weight inorganic compound such as BSH has low antigenicity because it has a low molecular weight and a small volume, or it is easy to ionize, and there has not been a measuring method or a measuring kit using a monoclonal antibody which can detect BSH with high sensitivity.

One of the objectives of the present invention resides in to provide a kit for measurement and an immunological measuring method for BSH which are highly sensitive and excellent in quantitative determination, using a monoclonal antibody or a fragment thereof which recognizes BSH with high sensitivity and high selectivity.

More specifically, one of the objectives of the present invention resides in to provide a measuring method and a kit thereof for BSH having excellent operability, especially utilizing a competitive immunoassay principle in simple and rapid measurement of BSH included in a bodily fluid.

### MEANS FOR SOLVING THE PROBLEM

The present inventors have been dedicated to carrying out a study, to find out that the measuring method and the measuring kit for BSH set forth below can accomplish the above-mentioned objectives, and completed the present invention.

In other words, the present invention provides a kit for immunoassay including an antibody-immobilized member wherein an anti-BSH monoclonal antibody or a fragment thereof is immobilized to a carrier, a diluent for a bodily fluid, and a conjugate of a hapten of BSH and a substance having discrimination capability.

The kit for immunoassay may include a known amount of a BSH reference standard.

The carrier may be a well plate.

The diluent for a bodily fluid may be a combination of a primary diluent and a secondary diluent for blood.

The present invention further provides a measuring method for BSH in a bodily fluid. The method includes: 1) a step of diluting a taken bodily fluid with a diluent for a bodily fluid, and contacting the dilution with an antibody-immobilized member wherein an anti-BSH monoclonal antibody or a fragment thereof is immobilized to a carrier in the presence of a conjugate of a happen of BSH and a substance having discrimination capability; and 2) a step of detecting the amount of an immunocomplex of BSH in the bodily fluid and the anti-BSH monoclonal antibody which can be formed by the contacting.

The carrier may be a well plate.
The bodily fluid may be blood, and the diluent for a bodily fluid may be a combination of a primary diluent and a secondary diluent for blood.

The step of detecting the amount of the immunocomplex may include a step of comparing the amount of discrimination label in a fluid obtained from the taken bodily fluid with the amount of discrimination label in a known amount of a BSH reference standard.

### EFFECT OF THE INVENTION

As described above, application of the present invention can provide a simple measuring kit or an immunoassay, method for BSH which has high selectivity for BSH included in a bodily fluid such as blood, and whish is capable of detecting BSH by distinguishing from BPA (borono-phenylalanine) and excellent in operability which does not require complicated steps.

### BRIEF DESCRIPTION OF THE DRAWINGS

[Fig. 1] An illustration showing a specific example of an antibody-immobilized member used for a measuring kit and a measuring method according to the present invention.
[Fig. 2] A standard curve of BSH measured with the measuring kit according to the present invention.
[Fig. 3] A graph showing the result of BSH measurement with the measuring kit according to the present invention.

### DESCRIPTION OF REFERENCE NUMERALS

1 Anti-BSH monoclonal antibody
2 Well plate
3 Blocking agent
4 Seal

### BEST MODES FOR CARRYING OUT THE INVENTION

Embodiments of the present invention will be described hereinafter.

The immunoassay method utilizing an antigen-antibody reaction is a measuring method which generally has high selectivity for a specific substance, and which is capable of highly-sensitive measurement and easy to operate. The present invention enables constructing of a measuring kit or a measuring method which can select means of the shape of the kit or an antibody involved in a reaction in measurement of BSH included in a bodily fluid such as blood, means of preparing a sample solution, means for reactant-to-reactant contact and operating means, having ease that operation optimal for the sample and a subject to be measured is possible, while utilizing a feature of an immunoassay method.

In the present invention, it has become possible to constitute a simple measuring kit excellent in operability and stability, and to carry out a measuring method, by preparing an antibody-immobilized member in which an anti-BSH monoclonal antibody or a fragment thereof has been previously immobilized to a carrier, a diluent for a bodily fluid, and a conjugate of a hapten of BSH contributing to the reaction and a substance which has discrimination capability (hereinafter sometimes referred to as a "discrimination conjugate"). In particular, a bodily fluid, which is a subject to be measured, may be diluted with a diluent for a bodily fluid, before a prepared sample is put into a reaction vessel in which an antibody-immobilized member wherein, for example, a discrimination conjugate and an anti-BSH monoclonal antibody or a fragment thereof is immobilized to a carrier (hereinafter sometimes simply referred to as an "antibody-immobilized member"), to allow reaction. Alternatively, when the antibody-immobilized member is a well plate, a Petri dish or the like, a diluted sample solution and a discrimination conjugate may be reacted in an antibody-immobilized member. Next, the concentration of BSH can be obtained by, for example, optically detecting a change dependent on the BSH concentration in a sample, so that a reactant thus obtained as a result of rapidly and accurately carrying out a competitive antigen-antibody reaction between BSH and a discrimination conjugate is detected as a chromogenic reaction derived from the discrimination conjugate bound to an antibody. As described above, the present invention has a feature of carrying out all operations such as reaction, measurement, or the like in a reaction system in which a diluent or a discrimination conjugate is previously included.

"Optically detect" herein refers to detection of a change in an antigen-antibody reaction retrieved as an optical change such as coloring or fluorescence, generated by the reaction itself, or coloring, fluorescence, turbidity or the like generated by addition of a chromogenic agent or a fluorescence agent, with an absorptiometer or the like.

In an immunoassay method, it is preferable to use a substance having a molecular structure similar to that of BSH and having an epitope of an antibody as a discrimination conjugate, so that the discrimination conjugate forms a competitive reaction with BSH, which is a subject to be measured. In the present invention, a discrimination conjugate is a conjugate of a derivative of BSH and a substance having discrimination capability, thereby allowing measurement which meets these conditions and has high selectivity. In addition, for an antibody, a monoclonal antibody is preferably used in view of high sensitivity and high selectivity, while a part of an antibody which has antigen-binding ability such as a Fab fragment, a F(ab')2 fragment or the like which is a variable part of a monoclonal antibody may also be preferably used. In the present invention, by combining these, it has become possible to provide a measuring kit for BSH using an immunoassay method with extremely high selectivity and high sensitivity. Here, a "substance having discrimination capability" refers to a substance or a carrier substance which directly or indirectly enables discrimination of results of an antigen-antibody reaction, and specifically includes carriers such as a biological material such as an enzyme or a fluorescent protein, a fluorescent substance having a low molecular weight, gold colloid, color latex beads and the like.

In the present invention, by a "bodily fluid", blood or lymph, a tissue fluid which fills an intercellular space outside of a blood vessel, and a body cavity fluid in a body cavity such as chorionic cavity fluid, pleural effusion, ascites, pericardial fluid, cerebrospinal fluid, synovial fluid, aqueous humor and the like are included. Furthermore, saliva, urine and the like are included. In the present invention, blood is especially preferably used as a subject to be tested.

A "carrier" of the present invention refers to, but is not limited specifically to, a well plate, beads, or a rod-like work or the like. As far as a resin to which a protein easily adsorbs such as polyethylene, polystyrene or the like is used as a material, the form is not limited specifically. A carrier which can easily contact with a solution and has a large surface area is preferable.

A diluent for a bodily fluid used in the present invention can be selected depending on the kind of a bodily fluid. Although not limited, when blood, which is especially preferably used in the present invention, is used as a subject to be tested, a phosphate buffer solution (phosphoric acid + sodium phosphate), phosphate buffered saline or the like, which is preferable to dilute blood, is prepared for dilution. In addition, for example, an acetate buffer solution (acetic acid + sodium acetate), a citrate buffer solution (citric acid + sodium citrate), a borate buffer solution, a tartrate buffer solution, a Tris buffer solution and the like can be mentioned.

In the present invention, the diluent for a bodily fluid can be a combination of a primary diluent and a secondary diluent. When the bodily fluid is blood, a combination of a primary diluent for hemolysis and a secondary diluent for mixing is preferably used. A phosphate buffer solution is preferably used as the primary diluent for hemolysis, and phosphate buffered saline is preferably used as the secondary diluent. As the dilution ratio, about ten- to five-hundred-fold in total is preferable, and it is possible to dilute a bodily fluid with a primary diluent at a certain degree of ratio, followed by further dilution with a secondary diluent immediately before use. Such combination is useful for preventing denaturation of the protein in a bodily fluid, while making a protein concentration capable of being detected just before a test.

In the measuring kit or the method of the present invention, since the result after an antigen-antibody reaction is detected as, for example, an optical change such as a change in a colorimetric quantity, it is preferable to make the criteria for the concentration of BSH in a sample solution clear. Then, since the amount of BSH included in a bodily fluid such as blood can be approximately grasped, it is able to carry out measurement excluding effects such as background or ambient temperature, by preparing a known amount of a BSH reference standard, preparing a standard curve under the same experimental conditions, and comparing a change in a colorimetric quantity with a reaction vessel. In other words, it will be able to provide a measuring kit or a method for BSH measurement using a simple and highly accurate immunoassay method, by carrying out comparison with a standard sharing measurement conditions.

In the present invention, detection of the result after an antigen-antibody reaction as a visual or optical change in a colorimetric quantity or the like can be carried out in a variety of modes depending on the kind of a carrier to which an antibody is immobilized, or the like. For example, when the antibody-immobilized member is a well plate, a reaction may be carried out within a well plate, followed by direct detection of the result of the reaction by an optical analysis for the well plate. For this purpose, a plate reader or the like can be preferably used. Alternatively, when a carrier to which an antibody is immobilized is beads, or other resin works or the like, the result of the reaction in a reaction vessel may also be detected by an optical analysis after transfer to another vessel.

### <Basic constitution of the measuring kit according to the present invention and the measuring method>

A measuring kit for BSH using the immunoassay method according to the present invention is **characterized in that** the kit includes (1) a diluent for a bodily fluid for diluting a taken bodily fluid, (2) a discrimination conjugate, and (3) an antibody-immobilized member to which an anti-BSH antibody or a fragment thereof is immobilized, and that a competitive antigen-antibody reaction is carried out.

As a measuring method for the above-mentioned measuring kit, among usual immunoassay methods, a known method, for example, an enzyme immunoassay, a gold colloid method or the like (Meth. Enzymol., 92, 147-523 (1983), Antibodies Vol. II IRL Press Oxford (1989)) can be applied.

Although a basic constitution of the measuring kit will be described hereinafter taking a case wherein, among other enzyme immunoassays, ELISA (Enzyme-Linked Immunosorbent Assay) of an antibody-solid phase type is applied as an example, the present invention is not limited thereto. In addition, materials or the like that will be described can be used commonly among, not limited to the measuring kit to which ELISA is applied, measuring kits using other labels, or measuring methods wherein ELISA or other labels are used. Here, the subject to be measured includes human blood.

### (1) Diluent for bodily fluid

A diluent for a bodily fluid is used for pretreatment of a bodily fluid such as blood, which is a subject to be measured. For example, it is a combination of a primary diluent for hemolysis and a secondary diluent for mixing, and a phosphate buffer solution is used as the primary diluent for hemolysis, and phosphate buffered saline is used as the secondary diluent. As the dilution ratio, about ten- to five-hundred-fold is preferable, and for example, it is possible to dilute a bodily fluid with a primary diluent twentyfold, and further dilute it with a secondary diluent tenfold just before use.

### (2) Preparation of discrimination conjugate

Since a discrimination conjugate wherein an enzyme is conjugated to BSH or a derivative thereof is used for a discrimination conjugate for carrying out an antigen-antibody reaction competitive with BSH, those having a ligand for the enzyme may be used as a BSH derivative. The enzyme which can be used includes, but is not limited specifically, so far as it is a publicly known enzyme, peroxidase, alkaline phosphatase, β-galactosidase and the like. Binding of the derivative and the enzyme may be carried out by any method, which is not limited specifically, so far as the enzyme would not be deactivated. The construction of a derivative of BSH will be specifically described hereinafter.
One of the BSH derivatives of the present invention is a compound having a structure represented by the following formula (1): 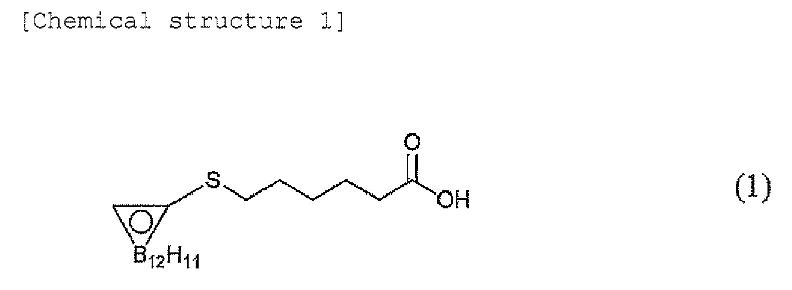 The above compound is 6-S-undecahydrododecaborylhexanoic acid and is used preferably as a BSH hapten. In the above formula (1), the carboxyl group is bound covalently to a high-molecular compound described later, to form a conjugate (complex).

Production of the above BSH hapten can be carried out by a known synthesis method and is not particularly limited. For example, the method shown in the following reaction scheme is preferably used because the compound can be obtained in high yield in each step. 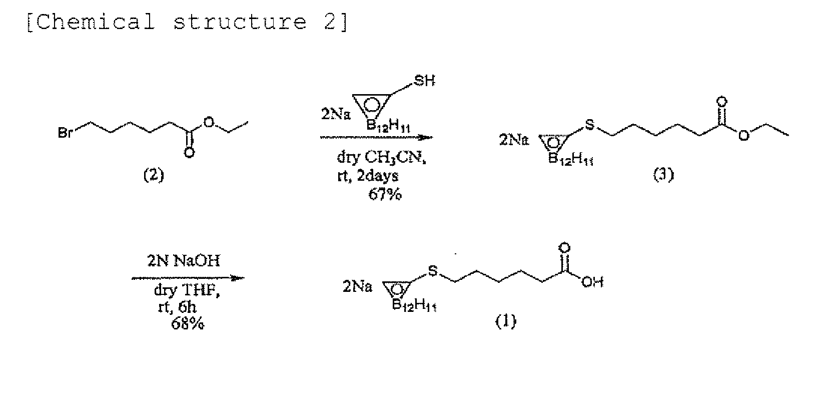 In the above reaction scheme, any starting compounds such as the compound of formula (2) and BSH are easily available compounds.

The synthesis method in the above each step will be described in detail in Example 1.

The above mercaptoundecahydrododecaborate (BSH) has an icosahedral boron cluster structure composed of boron, hydrogen and sulfur atoms. Although BSH is an inorganic low-molecular compound, BSH has a volume larger than that of a benzene ring, has so-called a three center bond structure wherein 3 boron atoms have 2 electrons in common, and is in a unique structure wherein electrons are localized. In the present invention, BSH is represented by the following formula (4) or (5):

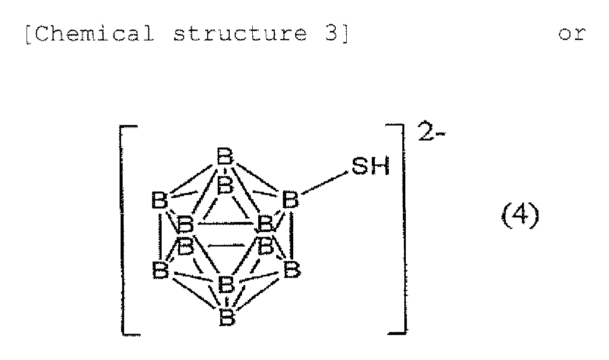

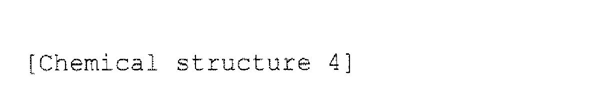

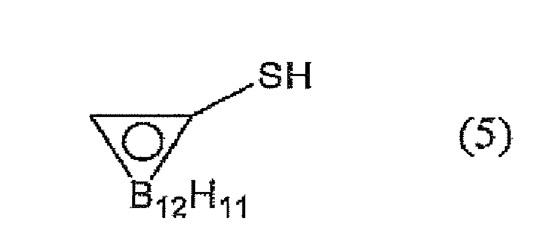

### (3) Preparation of immobilized member with an antibody (3-1) Preparation of antibody

The above BSH hapten is conjugated with a high-molecular compound (protein) such as bovine serum albumin (BSA), rabbit serum albumin (RSA), ovoalbumin (OVA), keyhole limpet hemocyanine (KLH), thyroglobulin (TG) and immunoglobulin and then used as immunogen.

The method of forming the conjugate can be carried out by a known method and is not particularly limited. For example, a carboxy group of the BSH hapten can be reacted with a functional group (for example, an amino group or the like) of the above high-molecular compound by a mixed acid anhydride method, an active ester method or the like to form the conjugate.

The antibody of the present invention can be prepared by the method in which a nonhuman animal such as rabbit, goat, or a mouse is immunized with the above conjugate by a conventional method, and then the resulting spleen cell is subjected to cell fusion with a myeloma cell to prepare a hybridoma cell which is then screened, and the resulting monoclonal antibody-producing hybridoma can be cultured to give the monoclonal antibody of the present invention (Current Protocols in Molecular Biology edit. Ausubel et al. (1987) Publish. John Wiley and Sons, Sections 11.4-11.11).

Preparation of the antibody can be carried out by a suitable combination of concentration and purification methods such as ultrafiltration, ammonium sulfate fractionation, ion-exchange chromatography, gel filtration chromatography and affinity chromatography.

Specifically, the above antibody can include, for example, an antibody having the following nucleotide sequences of heavy and light chains of a monoclonal antibody obtained in the Examples of the present invention or an antibody having the following amino acid sequences of heavy and light chains of a monoclonal antibody obtained in the Examples of the present invention. As long as the above nucleotide sequences or amino acid sequences exhibit the effect of the present invention, the sequences also include those that have a sequence in which a part of the above sequence is deleted, added, modified, substituted, or mutated. In this case, the homology between the above sequences and those that have a sequence in which a part of the above sequence is deleted, added, modified, substituted, or mutated is preferably 70% or more, more preferably 80% or more, still more preferably 90% or more and further more preferably 95% or more.

A sequence of heavy chain

(5'-)CTCGAGTCTGGCCCTGGAATATTGCAGCGCTCCCAGACCC
TCAGTCTGACTTGTTCTTTCTCTGGGTTTTCACTGAGCACTTCTGG
TATGGGTGTTGGCTGGTTTCGTCAGCCTTCAACAAAGGGTCTAGA
GTGGCTGGCAGACATTTGGTGGAATGACAATAAATACTATAATCC
ATCCCTGAAGAGCCGGCTCACAATCTCCAAGGATACCTCCAAAAA
CCAGGTATTCCTCAAGATCGCCAGTGTGGACACTATAGATACTGC
CACTTACTACTGTTCTCTAAGAAATAGTGCCGAAAAGACAAACAC
CTGGGGCCAAGGCACCACTCTCACAGTCTCCTCAGCCAAAACGAC
ACCCCCATCTGTCTATCCACTGGCCCCTGGATCTGCTGCCCAAAC
TAACTCCATGGTGACCCTGGGATGCCTGGTCAAGGGCTATTTCCC
TGAGCCAGTGACAGTGACCTGGAACTCTGGATCCCTGTCCAGCGG
TGTGCACACCTTCCCAGCTGTCCTGCAGTCTGACCTCTACACTCT
GAGCAGCTCAGTGACTGTCCCCTCCAGCACCTGGCCCAGCGAGAC
CGTCACCTGCAACGTTGCCCACCCGGCCAGCAGCACCAAGGTGGA
CAAGAAAATTGTGCCCAGGGATTGTACTAGT

A sequence of light chain

(5'−)GAGCTCGTTGTGACTCAGGAATCTGCACTCACCACATCAC
CTGGTGAAACAGTCACACTCACTTGTCGCTCAAGTACTGGGGCTG
TTACAACTAGTAACTATGTCAATTGGGTCCAAGAAAAACCAGATC
ATTTATTCACTGGTCTAATAGGTGGTACCAACAACCGAGCTCCAG
GTGTTCCTGCCAGATTCTCAGGCTCCCTGATTGGAGACAAGGCTG
CCCTCACCATCACAGGGGCACAGACTGAGGATGAGGCAATATATT
TCTGTGGTCTATGGTACAGCAACCATTGGGTGTTCGGTGGAGGAA
CCAAACTGACTGTCCTAGGCCAGCCCAAGTCTTCGCCATCAGTCA
CCCTGTTTCCACCTTCCTCTGAAGAGCTCGAGACTAACAAGGCCA

CACTGGTGTGTACGATCACTGATTTCTACCCAGGTGTGGTGACAG
TGGACTGGAAGGTAGATGGTACCCCTGTCACTCAGGGTATGGAGA
CAACCCAGCCTTCCAAACAGAGCAACAACAAGTACATGGCTAGC
AGCTACCTGACCCTGACAGCAAGAGCATGGGAAAGGCATAGCAG
TTACAGCTGCCAGGTCACTCATGAAGGTCACACTGTGGAGAAGAG
TTTGTCCCGTGCTGAGTGTTCCTAATTCTAGA

An amino acid sequence of heavy chain

(N−)LESGPGILQRSQTLSLTCSFSGFSLSTSGMGVGWFRQPSTKGL
EWLADIWWNDNKYYNPSLKSRLTISKDTSKNQVFLKIASVDTIDTAT
YYCSLRNSAEKTNTWGQGTTLTVSSAKTTPPSVYPLAPGSAAQTNS
MVTLGCLVKGYFPEPVTVTWNSGSLSSGVHTFPAVLQSDLYTLSSS
VTVPSSTWPSETVTCNVAHPASSTKVDKKIVPRDCTS

An amino acid sequence of light chain

(N—) ELVVTQESALTTSPGETVTLTCRSSTGAVTTSNYVNWVQEKP
DHLFTGLIGGTNNRAPGVPARFSGSLIGDKAALTITGAQTEDEAIYFC
GLWYSNHWVFGGGTKLTVLGQPKSSPSVTLFPPSSEELETNKATLV
CTITDFYPGVVTVDWKVDGTPVTQGMETTQPSKQSNNKYMASSYL
TLTARAWERHSSYSCQVTHEGHTVEKSLSRAECS

### (3-2) Carrier and antibody-immobilized member

As a carrier, a well plate or beads, which are easy to contact with a solution and have a part for immobilization of an antibody having a large surface area are preferably used. When a well pate is used, it is especially preferable, since the subsequent analysis can also be carried out within the well plate.

### (3-3) Immobilization of antibody to immobilization member

Immobilization of an antibody may be carried out by, for example, loading a buffer solution containing an antibody onto a sealable member, followed by incubation. The concentration of the antibody in the buffer solution is usually about 0.01 µg/mL to 10 µg/mL. As a buffer solution, without specific limitation, a publicly known buffer solution can be used.

### (3-4) Blocking of carrier surface

In order to prevent nonspecific adsorption of a contaminating substance in a sample to the carrier surface to affect the reaction, it is preferable to block a surface part onto which an antibody is not immobilized with a protein or the like which is not reactive to the antibody or a discrimination conjugate. As a blocking agent, bovine serum albumin (BSA) or a skim milk solution, or commercially available BlockAce or the like can be used. Blocking is carried out by contacting an antibody-immobilized member to a blocking agent solution of an excessive concentration and incubating the member overnight at, for example, about 4°C, followed by washing with a washing solution. A washing solution is not limited specifically, and, for example, a buffer solution containing a physiological salt concentration of sodium chloride can be used.

### (3-5) Stabilization of blocked immobilization member

The immobilization member on which an antibody is immobilized and blocked can be stabilized by drying. It is better to carry out drying at a low temperature, and the drying method may be any drying method such as vacuum freeze-drying, drying under reduced pressure, air drying or the like.

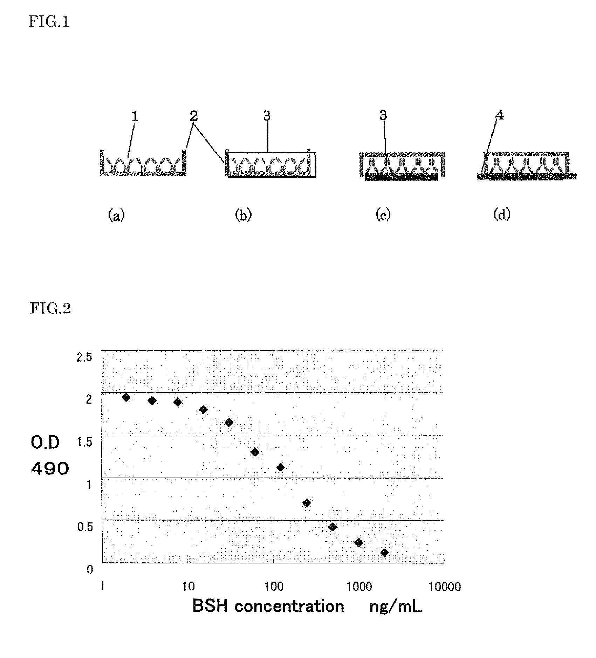

In particular, as exemplified in Fig. 1, the antibody-immobilized member may include a constitution wherein an antibody or a fragment thereof is immobilized within a plate (Fig. 1A), blocked (Fig. 1B), and subsequently dried (Fig. 1C). Especially, it is also possible to keep the antibody-immobilized member in a stable condition by sealing with a seal after drying (Fig. 1D) until just before use.

### (4) Antigen-antibody reaction

A sample prepared with a diluent for a bodily fluid to have an appropriate concentration is subjected to an antigen-antibody reaction. In particular, an antigen-antibody reaction is initiated by adding a discrimination conjugate and the diluted sample solution to a well plate as exemplified in Fig. 1. In addition, the BSH concentration in the sample can be accurately measured by additionally preparing a reaction system wherein a BSH reference standard and a discrimination conjugate are put into a well plate as exemplified in Fig. 1. It is better that an antigen-antibody reaction is carried out at a reaction temperature of 4°C to 37°C for a reaction time of about 5 minutes to 2 hours.

### (5) Detection

Next, the result of the reaction, a change dependent on the BSH concentration in the subject to be measured, is visually or optically detected. For example, when the carrier is a well plate, this detection can be carried out more rapidly and more simply by a direct analysis with a plate reader. Even in other cases, the result of the reaction can be detected by optically detecting directly the vessel used for the reaction.

More particularly, for example, after completion of the reaction, a substrate solution which develops color by the function of an enzyme of the discrimination conjugate bound to the immobilized antibody is added to the reaction vessel, after washing the well plate or the vessel used for the reaction with purified water, a buffer solution or the like. The chromogenic substrate is not limited specifically, so far as it is a publicly known substrate, and the absorbance may be measured using an enzyme, for example, in the case of peroxidase, 3,3',5,5'-tetramethylbenzidine. The concentration of BSH is determined by detecting the change in the colorimetric quantity generated after addition of the substrate solution. Other chromogenic substrates such as o-phenylenediamine may also be used. When alkaline phosphatase is used, for example, a method wherein p-nitrophenylphosphate is measured as a chromogenic substrate is included. In any of the chromogenic methods, the BSH concentration in the sample solution can be quantitatively determined using a calibration curve prepared on the basis of the relation between the absorbance and concentration of a reaction solution to which a known concentration of BSH is added.

In addition to enzyme immunoassay, BSH can be measured by an immunoassay method wherein a discrimination marker in the discrimination conjugate is substituted with color latex or gold colloid. These discrimination conjugates are prepared by binding a protein such as albumin to BSH or a derivative thereof, and solid-phasing this conjugate with the protein onto the surface of color latex or gold colloid using a publicly known method. BSH can be measured by carrying out an antigen-antibody reaction between the prepared discrimination conjugate and an immobilization member to which an antibody is immobilized, in the same manner as in the above-mentioned cases to which ELISA is applied. In the case of these immunoassay methods, without requiring subsequent chromogenic operation, color latex or gold colloid binds to the immobilized antibody on the surface of the immobilization member during an antigen-antibody reaction, to develop color. The BSH concentration can be measured by visually determining the degree of this color development.

A specific example of the measuring kit and the measuring method will be described hereinafter in a mode of examples, but the present invention is not limiter thereto.

### Example 1

### Preparation of antigen

In the following examples, an analysis, and separation and purification of a compound were carried out using the following models and reagents.
· NMR spectrum: JOEL, Ltd. JMTC-400/54/SS 400 MHz (manufactured by JOEL, Ltd.). Unless otherwise specified, TMS was used as an internal standard. In addition, the chemical shift set forth below is shown as a δ value.
· Silica gel for column chromatography: BW-200 (manufactured by Fuji Silysia Chemical Ltd.)

### (a) Synthesis of BSH-hexanoic acid ethyl ester

After dissolving BSH (101 mg, 0.46 mmol) in acetonitrile (10 mL), bromohexanoic acid ethyl ester (0.2 mL, 1.12 mmol) was slowly added dropwise to the solution with stirring at room temperature, and the solution was stirred at room temperature for 2 days. Acetonitrile was then removed by vacuum concentration, and the concentration residue was purified by silica gel column chromatography (chloroform : methanol = 9 : 1), to give a yellow oily matter (101 mg, yield: 67.4%).
· TLC: Rf = 0.58 (chloroform : methanol = 3 : 1)
· ¹H-NMR(DMSO) δ (ppm): 0.60-2.10(m), 1.08(t, 3H, J=7.08Hz), 1.34-1.42(m, 2H), 1.52-1.59(m, 2H), 1,63-1.70(m, 2H), 2.19(t, 2H, J=7.32Hz), 2.77(m, 2H), 3.95(q, 2H, J=7.08Hz)

### (b) Synthesis of BSH-hexanoic acid

The compound obtained in the above-mentioned (a) (113.7 mg, 0.31 mmol) was dissolved in methanol (2.0mL), and 2N sodium hydroxide (0.63 mL) was added dropwise to the solution. Thereafter, the solution was stirred at room temperature for 6 hours. After washing the reaction solution with ethyl acetate, 1N hydrochloric acid was added thereto until the pH became 3, and extraction was carried out with ethyl acetate. The extract was then washed with water and saturated saline and dried over magnesium sulfate, and the ethyl acetate was removed by vacuum concentration. The concentration residue was purified by silica gel column chromatography (chloroform : methanol = 4 : 1), to give a yellow oily matter (69.9 mg, yield: 67.4%).
· TLC : Rf = 0.30 (chloroform : methanol = 2 : 1)
• ¹H-NMR(DMSO) δ (ppm): 0.60-2.10(m), 1.30-1.37 (m, 2H), 1.45-1.53(m, 2H), 1,63-1.73(m,2H), 2.20(t, 2H, J=7.32Hz), 2.85(m, 2H)

### (c) Synthesis of BSH-hexanoic acid-BSA complex (immunogen using BSA)

Bovine serum albumin (11 mg, 164 nmol) and 760 µL of a borate buffer solution (sodium tetraborate 50 mmol, sodium chloride 15.4 mmol, sodium azide 0.3 mmol/pure water 100 mL), pH 9.4 was added to a Cent Tube, and the mixture was stirred overnight at 4°C, followed by addition of DMF (40 µL) (solution I).

The compound obtained in the above-mentioned (b) (6.0 mg, 18 µmol), N-hydroxysuccinimide (1.5 mg, 13.3 µmol), 1-ethyl-3-(3-dimethylaminopropyl)-carbodiimide hydrochloride (2.5 mg, 13.3 µmol), and DMF (200 µL) were added to another Cent Tube, and the mixture was stirred overnight at room temperature (solution II).

Solution II was added dropwise to Solution I (10 µL/5 minutes) and stirred for 2.5 hours at room temperature, followed by overnight stirring at 4 °C . This mixture was dialyzed against a 10% isopropanol-phosphate buffer solution (the buffer solution was replaced 6 times) for about 60 hours, to give a BSH-hexanoic acid-BSA complex (conjugate). After the boron concentration of this dialysate was measured by an ICP analysis, it was transferred to a 1.5 mL-Eppendorf tube and stored at 4°C.

### (d) Synthesis of BSH-hexanoic acid-KLH complex (immunogen using KLH)

As an immunogen, a conjugate of KLH and a BSH hapten of the present invention was prepared in the same manner as in the above-mentioned (c).

### Example 2

### Preparation of monoclonal antibody-producing hybridoma

### (a) Immunization of animal and preparation of antibody-producing cell

The BSH hapten-KLH conjugate synthesized in Example 1 was diluted with a phosphate buffer solution, pH 7.4 (PBS) (NaCl 137 mM, NaHPO₄·12H₂O 8.10 mM, HCl 2.68 mM, KH₂PO₄ 1.47 mM) to 500 µg/mL. Two milliliters of this antigen solution was mixed with RIBI Adjuvant System (RIBI/MPL^{™} + TDM Emulsion, R-700) warmed at 40°C for 10 minutes, and vortexed for 2 to 3 minutes, to be thoroughly mixed.

This mixture was subcutaneously injected to 5 Balb/c mice (8 weeks old, male) to immunize the animals (50 µg/dose). This procedure was carried out every 2 weeks, and blood drawing (orbital blood drawing) was carried out after one week from each immunization, starting from the second immunization. The drawn blood was collected in a 1.5 mL-tube and incubated at 37 °C for one hour, and left standing still overnight at 4°C.

### (b) Cell fusion

### (b-1) Preparation of DMEM medium

MilliQ was added to a pack of Dulbecco's modified Eagle medium (IWAKI DME/LOW, Lot. 99562013), 78.4 mg of gentamicin sulfate (gentamicin 642 µg/mg, SIGMA Lot. 105H0457), and 2.2 g of sodium bicarbonate (KANTO CHEMICAL CO., INC., Lot 302F1378) to make the total volume 1 L, and then the mixture was sterilized with a 0.22 µm-filter (MILLIPORE MILLEX^{™}-GV, 0.22 µm Filter SLGV01352), to prepare a DMEM medium.

### (b-2) Preparation of HAT medium and HT medium

Ten milliliters of sterilized MilliQ was added to a bottle of HAT supplement (SIGMA, Lot#61K8934), and the reagent in the bottle was completely dissolved. This solution was mixed in a 1/50 amount of a DMEM medium (15% FCS) and mixed therewith, to give a HAT medium. Ten milliliters of sterilized MilliQ was added to a bottle of HT supplement (SIGMA, Lot# 32K8928), and the reagent in the bottle was completely dissolved. The solution was mixed in a 1/50 amount of a DMEM medium (15% FCS) and mixed therewith, to give a HT medium.

### (b-3) Preparation of 50% PEG medium

Twenty grams of PEG6000 (PEG# 6000 (M. W. 7300 - 9000), NACALAI TESQUE, INC., Lot M8H2950) was added to 20 mL of a DMEM medium, and completely dissolved by stirring the mixture with a hot stirrer for 2 hours. The solution was then diluted to 40 mL, and sterilized using a 0.20 µm filter in a clean bench. Subsequently, the solution was dispensed by 1.8 mL and frozen, and dissolved just before use, followed by addition of 200 µL of DMSO (SIGMA, Lot# 42K2401), to prepare a 50% PEG (10% DMSO) solution.

### (b-4) Preparation of ACK lysis buffer solution

To 90 mL of MilliQ were added 802.3 mg of ammonium chloride, 10.01 mg of potassium bicarbonate, and 3.72 mg EDTA, and the solution was adjusted to pH 7.2 to 7.4 and diluted to 100 mL. This solution was put into a medium bottle and autoclaved at 121 °C for 20 minutes to be sterilized, to give an ACK lysis buffer solution (0.15 M ammonium chloride, 1.0 mM potassium bicarbonate, 0.1 mM EDTA, pH 7.2 to 7.4)

### (c) Preparation of spleen cell

Final immunization (tail vein injection) was carried out for a mouse of which antibody titer reached saturation, and after 3 days, its spleen was taken out. The spleen taken out was put into a Petri dish on ice containing DMEM, and the dish was placed in a clean bench. Unnecessary parts of the spleen taken out were removed, and the spleen was put into a 5-mL Petri dish containing a fresh DMEM medium. Spleen cells were scraped away using two pairs of tweezers, and unnecessary materials were removed by filtration with a cell strainer (FALCO-N2350, 70 µm nylon). Cells that passed through the strainer were collected in a glass centrifuging tube, and centrifuged at 1000 rpm for 10 minutes.

The supernatant was discarded, and 5 mL of an ACK lysis buffer solution was added to the tube and the mixture was homogenously mixed by pipetting. The mixture was left standing still for 5 minutes at room temperature, and the spleen cells were washed and removed (removal of erythrocytes). Twenty milliliters of a DMEM medium was added thereto and the mixture was centrifuged at 1000 rpm for 10 minutes, and the supernatant was removed. Additional 20 mL of a DMEM medium was added thereto, and cells were washed by pipetting and centrifuged at 1000 rpm for 10 minutes. The supernatant was removed, and 10 mL of a DMEM medium was added thereto and mixed by pipetting. Thereafter, the number of cells was counted using a hematocytometer (Erma Tokyo 4062).

### (d) Preparation of myeloma cells

Myeloma cells (P3X63Ag8U.1) were cultured by the day when cell fusion is carried out, and the cells were collected in a glass centrifuging tube just before use, and centrifuged at 1000 rpm for 10 minutes. The supernatant was removed, and 10 mL of a DMEM medium was added thereto and mixed by pipetting. Thereafter, the number of cells was counted using a hematocytometer (Erma Tokyo 4062).

### (e) Cell fusion

The amount of a myeloma cell suspension was adjusted to spleen cells : myeloma cells = 10 : 3, and the suspension was put into a centrifuging tube of a spleen cell suspension and mixed by pipetting. This suspension was centrifuged at 1000 rpm for 10 minutes, and the supernatant was removed. Thereafter, the supernatant was completely removed with a Pasteur pipette.

Subsequently, the centrifuging tube was beaten to spread the cells onto the wall of the centrifuging tube. The centrifuging tube was warmed with a hand, and 1 mL of a 50% PEG (10% DMSO) solution was gradually added thereto over 1 minute or more with turning the tube around. The centrifuging tube was turned around for 1 minute with warming, and 2 mL of a DMEM medium was added over 2 minutes or more in the same manner. Eight milliliters of a DMEM medium was further added thereto over 5 minutes or more.

Thereafter, the tube was centrifuged at 1000 rpm for 10 minutes, and the supernatant was discarded. Ten milliliters of a DMEM medium was further added thereto to wash cells, and the tube was centrifuged at 1000 rpm for 10 minutes. The supernatant was removed, and the cells were adjusted to have a cell density of 2 × 10⁵ cells/mL with a DMEM (15% FCS) medium. The cells were dispensed by 100 µL into a 96-well plate, and left standing still overnight at 37°C.

### (f) HAT selection

To the cells dispensed into the 96-well plate, about double amount of a HAT medium was dispensed by 100 µL, and the plate was left standing at 37°C for one week. Subsequently, 100 µL of a HAT medium was added thereto. Thereafter, hybridoma cells in a positive well were transferred to a 24-well plate, and a HT medium was added thereto to make the total amount 1 mL. Three days after, secondary screening was carried out, and a well which appeared to be positive was cloned by a limiting dilution method.

### (g) Cloning using a limiting dilution method

The number of cells of hybridoma cells in the well which appeared to be positive in the secondary screening was counted using a hematocytometer (Erma Tokyo 4062) to derive the concentration, and adjusted to 10 cells/mL or 5 cells/mL by serial dilution using a medium for cloning (20 mL of a DMEM (15% FCS) medium, 1 mL of Briclone). One-hundred microliters each of the dilution was dispensed into a 96 well plate, so that 1 or 0.5 cells were contained per well.

After the plate was left standing still at 37 °C for one week, a medium for cloning was added to each well by 100 µL, and the plate was left standing still at 37°C for another week. Cells in a well which appeared to be positive were transferred to a 48-well plate, and second cloning was carried out in the same manner, to finally give a single hybridoma cell. In the present invention, a hybridoma cell was prepared by the above-mentioned method, to establish multiple hybridoma strains including BSF-2. Among them, Hybridoma BSF-2 is internationally deposited with International Patent Organism Depositary, National Institute of Advanced Industrial Science and Technology (1-1-1 Higashi Tsukuba, Ibaraki 305-8566) on October 2, 2006, under the accession number BP-10689. In addition, BSF-2 is domestically deposited with International Patent Organism Depositary, National Institute of Advanced Industrial Science and Technology (1-1-1 Higashi Tsukuba, Ibaraki 305-8566) on February 22, 2006, under the accession number FERM P-20805.

### (h) Purification of antibody from serum-free medium

A hybridoma cell BSF-2 culture solution cultured in a serum-free medium was centrifuged at 1000 rpm for 10 minutes, and ammonium sulfate was added to the cell supernatant so as to have an ammonium sulfate concentration of 60%, to precipitate an antibody protein. This mixture was further stirred at 4°C for 2 hours, and further left standing overnight. Thereafter, the mixture was centrifuged at 13500 rpm at 4°C for 20 minutes, dissolved in a binding buffer, and dialyzed against the binding buffer.

After dialysis, the mixture was centrifuged at 8000 rpm for 10 minutes, and the supernatant was filtered with a 0.45 µm filter, to give a sample. After solution sending of 5 mL of ultrapure water to a column at a flow rate of 1 drop/second (1 to 2 mL/minute), 3 to 5 mL of a binding buffer was sent at a flow rate of 1 drop/second (1 to 2 mL/minute), to carry out equilibration of the column. Subsequently, the prepared sample was sent thereto at 1 drop/2 seconds (1 to 2 mL/minute), to adsorb the antibody to the column.

After unadsorbed components were removed by solution sending of 3 to 5 mL of a binding buffer (20 mM sodium phosphate, 0.8 M ammonium sulfate, pH 7.5) at a flow rate of 1 drop/second, 5 mL of an elution buffer (20 mM sodium phosphate, pH 7.5) was sent thereto at 1 drop/second (1 to 2 mL/minute), to elute the antibody. The eluate was collected by 0.5 mL.

The absorbance (OD = 280 nm) of all fractions was measured, and the concentration of the antibody protein was measured by a Bradford method for the fraction containing the antibody protein. Subsequently, the purity of the monoclonal antibody was confirmed by SDS-PAGE. In addition, a portion of the nucleotide sequence and a portion of the amino acid sequence of the obtained monoclonal antibody were same as described above. The type of the obtained monoclonal antibody was IgG, in particular, IgG1λ.

### Example 3

### Preparation of plate to which antibody is immobilized

A monoclonal antibody (a hybridoma strain (prepared from BSF-2) solution prepared to be 4 µg/mL using a PBS solution (phosphate buffer solution, pH 7.4) was dispensed into a 96-well plate for ELISA by 100 µL and left standing still at 37°C for one hour, thereby immobilizing the antibody to the plate. After the reaction, the plate was washed with a PBS-Tween solution (phosphate buffer solution (pH 7.4), 0.05% Tween 20), and 200 µl per well of a blocking solution (phosphate buffer solution (pH 7.4, 1% BSA) was added to the plate for preventing nonspecific adsorption. The well was sealed with a seal, and the reaction was carried out at 37°C for 2 hours, to carry out blocking.

Liquid in the plate was discarded, and remaining liquid was removed by banging the plate down onto Kimtowel. The plate was left standing still at 37°C for 2 hours, and dried by placing the plate with the bottom up. The plate was sealed with a seal and kept in cold storage. The dried plate thus obtained was confirmed not to deteriorate in cold storage at least for 4 months. The antibody-immobilized well plate thus obtained was used in the following Examples.

### Example 4

The kit for BSH measurement to which ELISA is applied in particular consists of the reagents exemplified in Table 1 below. First, a standard calibration curve of BSH was obtained using this measuring kit.

**[Table 1]**

| Constitution | Details of reagents |
|---|---|
| Label conjugate | Lyophilized horseradish peroxidase-conjugated BSH derivative prepared to 100 µg/mL |
| Antibody-immobilized well plate | Anti-BSH antibody was solid-phased, blocked, and thereafter dried |
| Diluent for bodily fluid | (Primary diluent) Fifty milligrams of Tween 20 was added to 100 mL of 25 mM phosphate buffer solution, and thereafter pH was adjusted to 7.5. (Secondary diluent) Ten microliters of label conjugate solution was added to a 25 mM phosphate buffer solution containing 0.05% Tween 20, and thereafter pH was adjusted to 7.2. Prepared by adding 1/20 amount of the primary diluent of control blood (tenfold). |

### (1) Preparation of standard solution

A BSH standard solution of 100 µg/mL was prepared with a PBS solution (0.5 µg/mL), and the solution was serially diluted with the primary diluent and the secondary diluent, to prepare a standard dilution series of 0, 2, 5, 10, 25, 50, 100, 200, 400, 800, 1000, and 2000 ng/mL.

### (2) Antigen-antibody reaction

The seal of the antibody-immobilized plate was removed, and the plate was washed with a plate washer (BIO-RAD Immnowasher). The label conjugate and each standard solution were mixed, and an antigen-antibody reaction was carried out at room temperature for 30 minutes.

### (3) Detection of change in colorimetric quantity

The plate was washed with a plate washer (BIO-RAD Immunowasher), to remove a discrimination conjugate which has not bound with the immobilized antibody. After thoroughly removing residual liquid of the washing solution, a PBS solution of an HRP-labeled competing agent (HPR-conjugated BSH-hexanoic acid) (0.5 µg/mL), which is a chromogenic reagent, was dispensed into the plate by 200 µl, and the plate was sealed with a seal. The plate was shaded and left standing still at room temperature for 10 minutes, and to a substrate solution (prepared by adding 8 mg of ODP to 20 mL of a phosphate citrate buffer solution (10 mM, pH 5.2) and adding 4 µl of H₂O₂ thereto) 50 µl each of a quenching solution (1N H₂SO₄) was added to quench the reaction. Thereafter, the absorbance at 490 nm was measured using a microplate reader (BIO-RAD/Model 550). The result is shown in Fig. 2. The x-axis represents the BSH concentration, and the y-axis represents the absorbance. As shown in Fig. 2, it was revealed that BSH can be specifically measured using a monoclonal antibody prepared from a hybridoma strain BSF-2 with an almost straight standard curve from 50 ng/mL to 500 ng/mL.

### Example 5

Next, a specific example of a measuring kit and a measuring method for measuring BSH in blood will be given. Each BSH measuring kit consists of the reagents as exemplified in Table 1. Measurement of BSH in blood was carried out using this measuring kit.

### (1) Preparation of sample solution

First, drawn blood was directly (whole blood) subjected to a test. EDTA·2Na was put into a 2 mg-test tube as an anticoagulant, and 2 mL of blood immediately after blood drawing was added thereto. The mixture was stirred with inverting the tube up and down. BSH was added to this blood, to prepare a 100 µg/mL BSH solution. After thoroughly mixing this diluent with a pipette using the primary diluent, the original blood was diluted twentyfold in a manner wherein a portion of the blood was taken out to another tube. Next, the foregoing primarily diluted sample solution was added to a tube into which the secondary diluent is put to dilute the solution tenfold, to prepare a sample for analysis diluted two-hundredfold. This sample was further diluted two-fold sequectially with a diluent containing blood, to prepare sample with various concentrations of BSH.

### (2) Antigen-antibody reaction

The sample solution was dispensed into the antibody-immobilized well plate by 100 µl in the presence of a discrimination conjugate, to carry out an antigen-antibody reaction at room temperature for 30 minutes.

### (3) Detection of change in colorimetric quantity

The plate was washed with a plate washer, to remove a discrimination conjugate which has not bound with the immobilized antibody. After thoroughly removing the residual liquid of the washing solution, a chromogenic reagent was added to the plate, and a chromogenic reaction was carried out for 10 minutes each. Thereafter, the absorbance at 490 nm was measured using a microplate reader (BIO-RAD/Model 550).

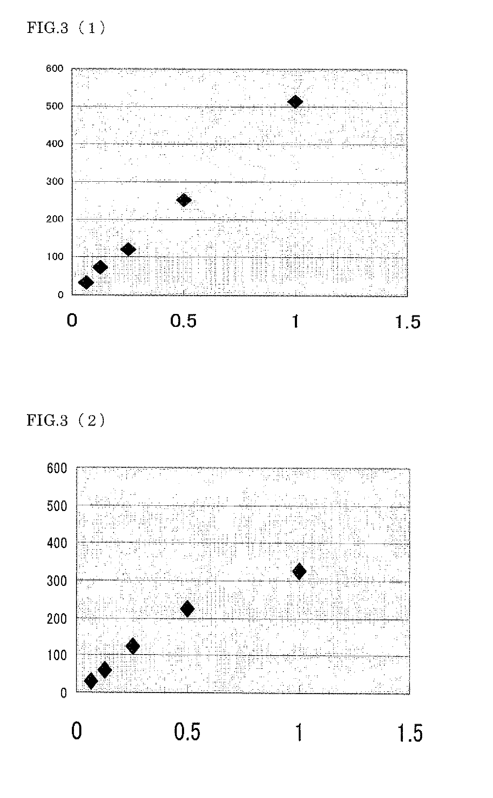

As a result, as shown in Fig. 3, for a blood sample, when blood was added to a diluent, a good straight line was obtained (Fig. 3 (1)). On the other hand, when only a buffer solution was used as a diluent, linearity is affected (Fig. 3 (2)). This showed that it is desirable to add blood upon preparation of a standard solution. In Fig. 3, the axis of abscissas represents the dilution ratio, and the axis of ordinate represents the BSH concentration (ng/mL).

### Example 6

Next, comparison between the measured value and a theoretical value of BSH in blood was carried out using a measuring kit of which reagents are exemplified in Table 1 in Example 4.

### (1) Preparation of sample solution

In the same manner as in Example 5, a sample for analysis of blood was prepared, so that the theoretical value of measurement of BSH was 500, 250, 100, and 50 ng/mL. Furthermore, a BPA-mixed sample was prepared by mixing BPA in the secondary diluent, at the same BSH concentration.

### (2) Antigen-antibody reaction

The sample solution was dispensed into the antibody-immobilized well plate by 100 µl in the presence of a discrimination conjugate, to carry out an antigen-antibody reaction at room temperature for 30 minutes.

### (3) Detection of change in colorimetric quantity

The plate was washed with a plate washer, to remove a discrimination conjugate which has not bound with the immobilized antibody. After thoroughly removing the residual liquid of the washing solution, a chromogenic reagent was added to the plate, to carry out a chromogenic reaction for each 10 minutes. Thereafter, the absorbance at 490 nm was measured using a microplate reader (BIO-RAD/Model 550).

As a result, as shown in Table 2, the higher the concentration of BSH was, the better result of an analysis was obtained.

**[Table 2]**

| BSH concentration µg/mL | Theoretical value at the time of measurement ng/mL | Measured value | | | | | Addition recovery ratio (%) | CV (%) |
|---|---|---|---|---|---|---|---|---|
| 100 | 500 | 525 | 500 | 423 | 518 | 471 | 85-105 | 9 |
| 50 | 250 | 271 | 255 | 277 | 274 | 277 | 102-111 | 3 |
| 20 | 100 | 121 | 112 | 83 | 111 | 109 | 83-121 | 13 |
| 10 | 50 | 51 | 49 | 30 | 51 | 48 | 60-102 | 19 |

On the other hand, as shown in Table 3, a good analysis result was obtained similarly in the presence of BPA.

**[Table 3]**

| BSH concentration | Theoretical value | BPA addition | Without addition |
|---|---|---|---|
| 100 µg/mL | 500 | 467 | 471 |
| 50 | 250 | 263 | 277 |
| 20 | 100 | 108 | 109 |
| 10 | 50 | 47 | 48 |

Although the measurement kit for detecting the concentration in bodily fluid of BSH by ELISA has been described herein above based on a specific constitutional example, it is obvious that the present invention is not limited to these examples.

## Claims

1. A kit for immunoassay, comprising an antibody-immobilized member wherein an anti-BSH monoclonal antibody or a fragment thereof is immobilized to a carrier, a diluent for a bodily fluid, and a conjugate of a hapten of BSH and a substance having discrimination capability.

2. The kit for immunoassay according to claim 1, further comprising a known amount of a BSH reference standard.

3. The kit for immunoassay according to claim 1 or 2, wherein said carrier is a well plate.

4. The kit for immunoassay according to any of claims 1 to 3, wherein said diluent for a bodily fluid is a combination of a primary diluent and a secondary diluent for blood.

5. A measuring method for BSH in a bodily fluid, comprising
1) a step of diluting a taken bodily fluid with a diluent for a bodily fluid, and contacting the obtained diluted sample solution with an antibody-immobilized member wherein an anti-BSH monoclonal antibody or a fragment thereof is immobilized to a carrier, in the presence of a conjugate of a hapten of BSH and a substance having discrimination capability; and
2) a step of detecting the amount of an immunocomplex of BSH in the bodily fluid and the anti-BSH monoclonal antibody or the fragment thereof, which can be formed by said contacting.

6. The measuring method according to claim 5, wherein said carrier is a well plate.

7. The measuring method according to claim 5 or 6, wherein said bodily fluid is blood, and wherein said diluent for a bodily fluid is a combination of a primary diluent and a secondary diluent for blood.

8. The measuring method according to any of claims 5 to 7, wherein said step of detecting the amount of the immunocomplex includes a step of comparing the amount of discrimination label in a diluted sample solution obtained from said taken bodily fluid with the amount of discrimination label in a known amount of a BSH reference standard.
